# EUROPEAN PATENT APPLICATION

(11) **EP 0 956 870 A1**
(43) Date of publication of application: **17.11.1999**
(21) Application number: 98924623.6
(22) Date of filing: 15.06.1998
(51) Int. Cl.: A61L 33/00

(54) **ANTITHROMBOTIC MEDICAL MATERIAL**

(30) Priority: 18.06.1997 JP 16149497
(71) Applicant: TORAY INDUSTRIES, INC., Tokyo 103-8666 (JP)
(72) Inventor: OYAMA, Yoshihiro, Otsu-shi, Shiga 520-0843 (JP); INOUE, Satoru, Otsu-shi, Shiga 520-0842 (JP)
(74) Representative: Coleiro, Raymond
(86) International application number: JP9802626
(87) International publication number: WO9857679

(57) **Abstract**

The antithrombotic medical material of the present invention is a medical material having, on the surface, a coating comprising graft copolymer in which monomer containing a quaternary ammonium group and hydrophilic monomer are grafted to a hydrophobic polymer, and an anticoagulant such as heparin is fixed to a depth of at least 45% from the coating surface and, furthermore, the film thickness in which anticoagulant has been fixed is at least 3 µm.

The antithrombotic medical material of this invention provides an antithrombotic medical material which is outstanding in its antithrombotic character and has a fixed product quality, in particular discoloration of the coating does not occur.

## Description

### Technical Field

The present invention relates to a medical material with outstanding antithrombotic character and, in particular, it relates to an antithrombotic medical material which is preferably used at the inner or outer face of a medical device which directly contacts the blood or body fluids and tissues.

### Technical Background

When blood comes into contact with a medical material, blood coagulation occurs at the surface thereof and a blood clot is formed. In the case where a treatment or a measurement is carried out using a medical device, due to the influence of this blood clot delivery of a drug is made impossible or the measurement sensitivity is lowered. Furthermore, a blood clot stops blood flow or moves along with the blood causing complications such as pulmonary thrombosis, cerebral thrombosis and myocardial infarction.

Consequently, in using such a medical material in practice, an anticoagulant like heparin, futhan or coumarin is administered and clot formation prevented. However, when an anticoagulant such as heparin is generally administered, there is the great disadvantage that the danger of haemorrhage is considerably increased.

Hence, if the surface of the medical material is rendered antithrombotic with a heparinized material, then it is possible to carry out treatment or diagnosis safely.

Such heparinized antithrombotic materials can broadly be divided into three.

Firstly, there are the materials formed simply by mixing heparin (normally in the form of heparin sodium) into a material. Such materials have the disadvantage that, while in contact with the blood, the heparin is washed out into the blood and the antithrombotic properties disappear in a short time.

Secondly, there are those in which covalent bonding is performed with the material, utilizing the hydroxyl, carboxyl or amino groups of the heparin. For example, there is known the method where heparin and an antithrombin are jointly present and fixed on a support, or fixed to fibroin using a coupling agent. Such materials suffer from the problem that their synthesis is complex and the antithrombotic character of the heparin is reduced.

The third material is one in which ionic bonding is effected between ionic residual groups in the heparin and cationic residual groups in the material. For example, in Japanese Examined Patent Publication No. 6-24592 and Japanese Examined Patent Publication No. 6-24593, ionic-bonded complexes of heparin and a polymerizable basic compound having a quaternary ammonium salt are disclosed. Such a material has the disadvantage that the setting of conditions for polymerization of the ionic-bonded complex is difficult and discoloration of the material tends to occur, so obtaining an antithrombotic medical material of uniform product quality is difficult. Again, in Japanese Unexamined Patent Publication No. 57-14358 there is disclosed a medical material having a coating comprising a graft copolymer in which monomer containing a quaternary ammonium group and hydrophilic monomer are grafted to the surface of polyvinyl chloride. Such a material has an excellent antithrombotic character but it is still inadequate and a higher level of antithrombotic character has been demanded.

The present invention aims to resolve such difficulties of the prior art, and has the objective of providing an antithrombotic medical material with outstanding antithrombotic character and of uniform product quality, in particular one where discoloration of the coating does not occur.

### Disclosure of the Invention

The antithrombotic medical material of the present invention is composed as follows. That is to say, it is an antithrombotic medical material characterized in that it is a medical material having, on a substrate surface, a coating of a graft copolymer in which monomer containing a quaternary ammonium group and hydrophilic monomer are grafted to a hydrophobic polymer, and an anticoagulant is fixed to a depth of at least 45% from said coating surface and, furthermore, the film thickness in which said anticoagulant has been fixed is at least 3 µm.

In the present invention, it is possible to cite the following preferred embodiments.
(1) That in which the aforesaid anticoagulant is heparin.
(2) That in which the aforesaid hydrophobic polymer is polyvinyl chloride.
(3) That in which the aforesaid quaternary ammonium group-containing monomer is a vinyl compound.
(4) That in which the aforesaid hydrophilic monomer is a vinyl compound.
(5) That in which the aforesaid quaternary ammonium group-containing monomer is an acrylic acid derivative represented by general formula [I].

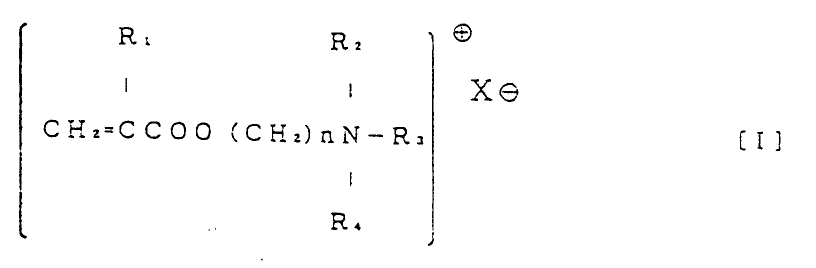

(R₁ is H or CH₃; R₂, R₃ and R₄ are respectively H or a C₁ to C₃ alkyl group; X is a negative atomic grouping which can form a salt with amine nitrogen and n = 2-6.)

(5) That in which the aforesaid hydrophilic monomer is an acrylic acid derivative represented by general formula [II].

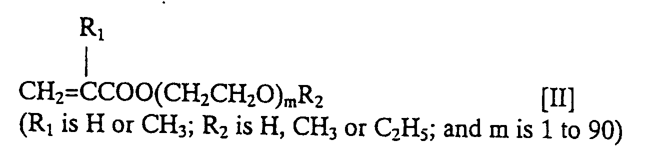

### Brief Explanation of the Drawing

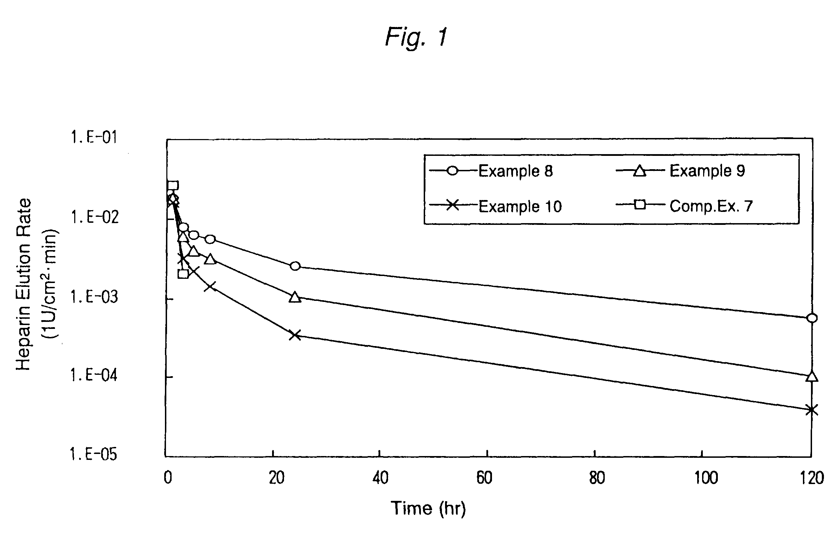

Figure 1 is a graph showing the heparin elution rate results obtained in Example 8 to Example 10 and in Comparative Example 7.

### Optimum Configuration for Practising the Invention

In the antithrombotic medical material of the present invention, it is necessary that an anticoagulant be fixed to a depth of at least 45% from the surface of the coating of graft copolymer in which monomer containing a quaternary ammonium group and hydrophilic monomer are grafted to a hydrophobic polymer and, furthermore, the film thickness in which said anticoagulant has been fixed will be at least 3 µm. In particular, it is preferred that the anticoagulant be fixed to a depth of at least 75% and it is preferred that the film thickness in which said anticoagulant has been fixed is from 6 µm to 65 µm or above. If the fixed depth of anticoagulant is less than 45% then the coating will tend to discolour and maintaining the product quality will be difficult. Again, if the film thickness in which anticoagulant has been fixed is less than 3 µm, then the antithrombotic properties are inadequate, while if it exceeds 100 µm then control of the film thickness becomes difficult. The film thickness as an antithrombotic medical material is in practice no more than 100mµ and preferably no more than 65mµ.

As examples of the hydrophobic polymer employed in the present invention, there are the polymers of vinyl chloride, methyl methacrylate, styrene, acrylonitrile, vinyl acetate and glycidyl methacrylate, and copolymers in which these are the chief component, but, from amongst these, polyvinyl chloride is particularly preferably used on account of the fact that it may have various levels of hardness and has outstanding mechanical strength.

Furthermore, as examples of the monomer containing a quaternary ammonium group employed in the present invention, there are the quaternary ammonium salts of vinyl pyridine and derivatives thereof, dimethyl diallyl ammonium halide, diethyl diallyl ammonium halide and the like but, from amongst these, vinyl compounds with a quaternary ammonium group are preferably employed.

There is particularly preferably used, as the vinyl compound with a quaternary ammonium group, an acrylic acid derivative represented by general formula [I].

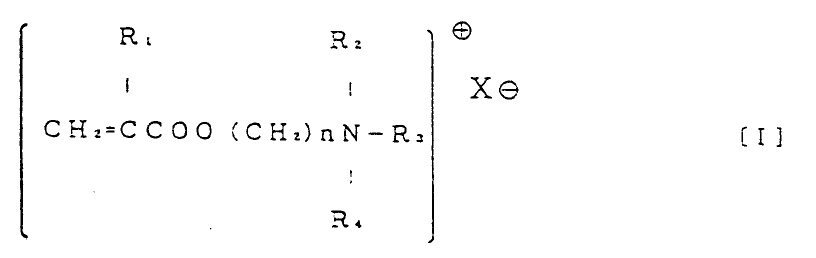

(R₁ is H or CH₃; R₂, R₃ and R₄ are respectively H or a C₁ to C₃ alkyl group; X is a negative atomic grouping which can form a salt with amine nitrogen and n = 2-6.)

Specific examples of the acrylic acid derivatives represented by general formula [I] are the quaternary salts of diethylaminoethyl methacrylate, dimethyl-aminoethyl methacrylate and diethylaminopropyl methacrylate, and from the point of view of cost and ready availability the quaternary salts of diethyl-aminoethyl methacrylate are particularly preferably used.

Furthermore, as examples of the hydrophilic monomer used in the present invention there are hydrophilic group-containing vinyl compounds, divinyl compounds, cyclic ether compounds and cyclic imine compounds but, from amongst these, the vinyl compounds with a hydrophilic group are preferably used.

Acrylic acid derivatives represented by general formula [II] are particularly preferably used as the vinyl compound with a hydrophilic group.

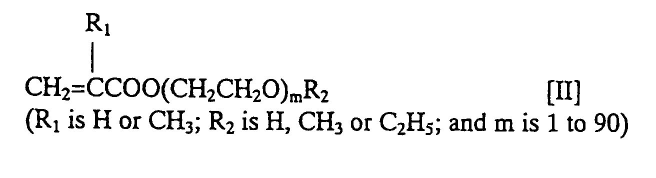

Specific examples of the acrylic acid derivatives represented by general formula [II] are methoxy-polyethyleneglycol methacrylate, methoxypolyethyleneglycol acrylate, polyethyleneglycol methacrylate and the like and, in particular, methoxypolyethyleneglycol methacrylate is preferably employed. As well as being hydrophilic, these compounds are characterized by having a considerable copolymerization ability.

Preferably, an acrylic acid derivative represented by general formula [I] is used as the monomer containing a quaternary ammonium group and an acrylic acid derivative represented by general formula [II] as the hydrophilic monomer.

When an acrylic acid derivative of general formula [I] and an acrylic acid derivative of general formula [II] are simultaneously subjected to graft polymerization, the graft polymerization of general formula [I] is promoted and, moreover, the hydrophilicity of the graft polymer obtained is enhanced and an anticoagulant such as heparin is readily taken-in within the polymer chains, so there is shown the effect of an increased antithrombotic character. Furthermore, the polymer flexibility is increased and fabrication facilitated and, again, there is also conferred the outstanding characteristic that even when used over a long period there is no harm to the blood vessel interior or to the tissues.

Taken together, the content in the polymer of the acrylic acid derivatives represented by general formulae [I] and [II] is preferably from 1 to 60 wt% and more preferably from 10 to 50 wt%. Furthermore, where the respective contents of the acrylic acid derivative represented by general formula [I] and of the acrylic acid derivative represented by general formula [II] lie within the range 0.5 to 40 wt%, this is particularly preferred, and it is especially desirable that the employed ratio of the former to the latter be 1 : 2.

With graft polymer having such a percentage grafting and having this proportion of acrylic acid derivatives represented by general formulae [I] and [II] during the grafting, there is the effect that, while the mechanical properties of the hydrophobic polymer such as strength and flexibility are still maintained, there are also contributed the various characteristics which should be provided as a medical material.

In the present invention, the method for producing the graft copolymer is not particularly restricted but, for example, it can be obtained by subjecting the hydrophobic polymer to a graft activation treatment, then adding the hydrophilic monomers and carrying out polymerization by a suitable polymerization method.

In the case for example where vinyl chloride is employed as the hydrophobic polymer there is preferably used, as the graft activation treatment method, a method such as replacing chlorine atoms by means of dithiocarbamate groups at which radicals are readily generated by light irradiation or the like. This method is conducted by dissolving the vinyl chloride polymer in a suitable organic solvent such as dimethylformamide or dimethylsulphoxide and then performing reaction between the polymer and an alkali metal dialkyldithiocarbamate at 30-70°C, such that from 1 to 10% of the chlorine atoms are replaced by dithiocarbamate groups.

Again, there may be employed as the polymerization method a method such as photopolymerization, thermal polymerization or radiation polymerization, from amongst which methods photopolymerization is preferably used. A high pressure mercury lamp will suffice as the light source. An aforesaid organic solvent is suitably employed as the solvent. Appropriately, the concentration of the vinyl chloride and vinyl compounds will preferably respectively be from 2 to 20 wt% and more preferably from about 5 to 10 wt%. The polymerization temperature is preferably from 0 to 40°C and in particular from 10 to 30°C.

In the present invention, when producing the copolymer containing the quaternary salt of a tertiary amino group, there is no restriction to using a quaternized component as the copolymer component and there may also be employed a method whereby the monomer containing said tertiary amino group is first polymerized, after which the polymer is then subjected to the action of a quaternizing agent, either in solution or at its surface following fabrication while still in the tertiary amino group form. In particular, in the case of the method of quaternizing following fabrication, the handling of the polymer solution is easy and it is a simple method, so this is preferred, Next, a more detailed explanation is given of the method of quaternizing following fabrication.

In practising the present invention, the aforesaid graft copolymer can preferably be employed by coating the catheter or other such substrate material which comes into direct contact with the blood or body fluids and tissues, using a common solvent for the substrate and copolymer. Here, as the common solvent for the substrate material and copolymer, there can be used for example dimethylformamide, dimethylacetamide, dimethylsulphoxide, cyclohexanone, methyl isobutyl ketone, tetrahydrofuran, chloroform or solvent mixtures of these, but, from the point of view of ease of fabrication, one with a boiling point of around 30 to 80°C such as tetrahydrofuran is preferred. A polymer material such as polyurethane or polyvinyl chloride is used as the substrate.

In the present invention, the thickness of the coating comprising the copolymer will differ according to the copolymer concentration in the coating solution (from 1 to 50 wt% is preferred) and the number of times coating is conducted but, in order to manifest an effective antithrombotic character within the body over a long period, there is preferably employed a coating of thickness at least 3 µm and more preferably at least 5 µm. The upper limit of coating thickness is not particularly restricted but it is normally about 100 µm.

The substrate which has been coated in this way is then dried. With regard to the drying method, this is preferably carried out in air or in an inert gas atmosphere such as nitrogen or argon, and the drying temperature will differ depending on the type of solvent employed but a temperature in the range from room temperature to 100°C is suitable.

Next, the quaternizing reaction is carried out and this quaternizing reaction can be performed by dissolving the quaternizing agent in an organic solvent such as methanol or acetone, and adding water to produce a mixed solvent, then introducing therein the aforesaid coated and dried substrate such as a medical moulded article. There is used a known quaternizing agent such as an alkyl halide like ethyl bromide, or hydrogen bromide, monochloroacetic acid or the like as the quaternizing agent. Here, there is preferably employed from 5 to 90% and in particular from 20 to 50% of the organic solvent in terms of the water. Again, the reaction temperature is preferably from 10 to 70°C and more preferably from 40 to 60°C.

The fixing of the anticoagulant in the coating of the coated copolymer thus obtained is carried out by bringing the coated copolymer coating into contact with an aqueous solution of the anticoagulant. The anticoagulant employed in the present invention is appropriately a compound having minus ions which bond with the quaternary ammonium, specific examples being heparin, fragmin or the like, or the salts thereof, but heparin or a salt thereof is preferably employed.

The heparin or salt thereof employed here is not particularly restricted but, normally, there is used heparin sodium or benzalkonium heparin.

The fixing of the anticoagulant such as heparin in the copolymer coating can be carried out by immersion of the copolymer coating for from several hours up to 5 days at 70-80°C in an aqueous solution containing preferably at least 0.5 wt% and more preferably from 0.5 to 5 wt% of the anticoagulant. As the temperature is raised, there is exhibited a tendency for the percentage fixed with heparin (the thickness of the fixed layer) to increase. Hitherto, because of concerns such as (1) the deactivation of the heparin by high temperatures, (2) cracks in the coating layer or (3) deterioration or dimensional distortion of the substrate, the raising of the heparin fixing temperature has not been fully investigated. In the case where heparin is used as the anticoagulation agent, it is preferred that from 0 to 0.3 N sodium chloride be included in the aqueous heparin solution.

Following the completion of the fixing of the anti-coagulant, the substrate is dried under vacuum at normal temperature, then sterilizing performed with ethylene oxide gas (EOG gas) or the like, after which an EOG gas removal operation is carried out for 24 hours at 60°C.

The percentage fixed anticoagulant in the coating at the medical material surface produced by the above method has been determined by producing a slice of the material cross-section, then staining this with toluidine blue or other such thiazine dye, and measuring the percentage (A/B x 100) from the film thickness of the stained portion (film thickness of fixed heparin; A) and the film thickness of the graft copolymer (B). Again, with regard to the discoloration of the coating, this was observed by eye. The evaluation of the antithrombotic character was carried by an in vitro evaluation employing fresh rabbit blood and by measuring the heparin elution rate from a fixed-with-heparin tube.

As a result of these evaluations, it has been shown that a medical material based on the present invention has extremely outstanding antithrombotic character and, at the same time, there is absolutely no discoloration of the coating.

The antithrombotic medical material obtained in this way can preferably be used for the interior or exterior faces of medical devices which come into direct contact with the blood. As examples of the medical devices there are catheters, multilumen catheters, drip chamber filter meshes for blood circuits employed for extracorporeal circulation, sheath introducers, canulas, guide wires, by-pass tubes, artificial hearts, artificial heart valves, extracorporeal circulation circuits for assisted circulation devices, AV shunts for artificial kidneys artificial blood vessels, film or hollow fibre dialysis membranes for artificial kidneys, film or hollow fibre oxygen-exchanging membranes for artificial lungs, and connectors for tube connection. In particular, it can be used preferably for coating of the inner or outer faces of catheters such as multilumen catheters, sheath introducers and the drip chamber filter meshes of blood circuits for extracorporeal circulation.

### Examples

Below, the invention is explained in further detail by means of examples.

### Example 1

120 g of polyvinyl chloride of degree of polymerization 1,100 was dissolved in 2 litres of dimethylformamide, 2.704 g of sodium diethyldithiocarbamate then added and reaction carried out for 3 hours at 50°C, after which the polymer was reprecipitated in methanol and dried and, in this way, polyvinyl chloride rendered active to photo-grafting was obtained (hereinafter abbreviated to DTC-modified polyvinyl chloride).

80 g of this DTC-modified polyvinyl chloride was dissolved in 1250 ml of tetrahydrofuran, then 200 g of methoxypolyethyleneglycol methacrylate (degree of polymerization of the polyethylene glycol moiety = 20-23) and 80 g of dimethylaminoethyl methacrylate were added and photo-graft polymerization carried out by irradiating for 9.5 hours at 30°C with a 100 W high pressure mercury lamp (Ushio Denki UM-102) in a light source interior immersion type photo-reaction device.

The composition, by weight ratio, of the graft copolymer was 54% vinyl chloride, 30% methoxypolyethyleneglycol methacrylate and 16% dimethylaminoethyl methacrylate.

Next, with this graft copolymer as a 11 wt% solution in tetrahydrofuran, coating was conducted of the exterior face of a polyurethane tube (length 70 cm and outer diameter 1.6 mm). After leaving the coated tube for 24 hours at room temperature, it was cut to 7 cm. The thickness of the coating layer at this time was 37.5 µm.

After the completion of the drying, the coated tube and 50 ml of quaternizing agent (a solution obtained by adding 10 ml of ethyl bromide to 300 ml of methanol and then adding 700 ml of water and stirring) were introduced into a glass tube of diameter 25 mm and length 20 cm, and reaction carried out for 1 hour at 50°C. The quaternizing agent was then replaced with a 1/3 solution of methanol/water and extraction of the unreacted ethyl bromide and the like carried out twice at 60°C. After washing with water, washing was carried out with 0.1 N salt solution, then a 0.1 N saline solution of 0.69 wt% heparin was added and a heparinizing reaction carried out for 22.5 hours at 80°C. The result of a visual assessment for cracking and separation of the coating at this time is shown at the right side of Table 1.

Following the end of the reaction, the uncoupled heparin and residual salts were removed with water at 60°C and drying performed for 24 hours under vacuum. This tube was then cut in two, with one piece being used as the sample for measurement of the percentage fixed with heparin from the coating surface and the other being used as the sample for evaluation of the discoloration of the coating.

The measurement of the percentage fixed with heparin was carried out as follows. Specifically, the tube was subjected to circular slicing to produce a thin slice of no more than 0.5 mm and this was placed on a slide glass. On top, there was added 0.02% toluidine blue aqueous solution and this was left for 10 minutes. The toluidine blue solution was then soaked up with filter paper and the cross-section of the sample observed under a microscope and the film thickness measured. The film thickness stained by the toluidine blue (the thickness of film fixed with heparin; referred to as A) and the film thickness of the graft copolymer (referred to as B) were obtained, and the percentage fixed with heparin calculated (A/B x 100). The results are shown in Table 1.

On the other hand, with regard to the discoloration of the coating, after placing in a sterilizing bag, EOG gas sterilization was carried out for 7 hours at 42°C, after which heating was performed for 14 hours at 60°C. Table 1 shows the result as to whether or not there was discoloration.

### Comparative Example 1

The same method was conducted as in Example 1 excepting that the heparinizing reaction was carried out at 60°C. The percentage fixed with heparin, whether or not there was discoloration of the coating and whether or not there was cracking/separation of the coating were determined, and the results are shown in Table 1.

### Example 2

The same method was conducted as in Example 1 excepting that the quaternizing reaction time in Example 1 was made 4 hours and the heparinizing reaction was carried out at 70°C for 15 hours. Measurement of the percentage fixed with heparin, and the evaluation as to whether or not there was discoloration of the coating and whether or not there was cracking/separation of the coating were carried out, and the results are shown in Table 1.

### Comparative Example 2

The same method was conducted as in Example 2 excepting that the heparinizing reaction was carried out at 60°C. The percentage fixed with heparin, whether or not there was discoloration of the coating and whether or not there was cracking/separation of the coating were determined, and the results are shown in Table 1.

### Example 3

The same method was conducted as in Example 2 excepting that the graft copolymer was used as a 9 wt% solution in tetrahydrofuran, and the coating carried out employing this. Measurement of the percentage fixed with heparin, and evaluation as to whether or not there was discoloration of the coating and whether or not there was cracking/ separation of the coating were carried out, and the results are shown in Table 1.

### Comparative Example 3

The same method was conducted as in Example 3 excepting that the heparinizing reaction was carried out at a temperature of 60°C. The percentage fixed with heparin, whether or not there was discoloration of the coating and whether or not there was cracking/separation of the coating were determined, and the results are shown in Table 1.

### Comparative Example 4

The same method was conducted as in Example 1 excepting that no heparinizing reaction was carried out. Measurement of the percentage fixed with heparin, and evaluation as to whether or not there was discoloration of the coating and whether or not there was cracking/ separation of the coating were carried out, and the results are shown in Table 1.

### Example 4

With the graft copolymer of Example 1 as a 9 wt% solution in tetrahydrofuran, coating was carried out of the inner face of a TYGON tube (produced by the Norton Co., length 50 cm and internal diameter 12.5 mm) and then this was dried for 24 hours at room temperature. Following the completion of drying, the coated tube and the total quantity of quaternizing agent (a solution obtained by adding 20 ml of ethyl bromide to 600 ml of methanol, then further adding 1400 ml of water and stirring) were introduced into a glass tube of diameter 40 mm and length 1 m) and reaction carried out for 1 hour at 50°C. The quaternizing agent was then replaced with a 1/3 solution of methanol/water and unreacted ethyl bromide and the like extracted twice at 60°C. After washing with water, washing was performed with 0.1 N saline solution and then a 0.1 N saline solution of 0.75 wt% heparin was added and the heparinizing reaction carried out at 80°C for 16 hours. The result of a visual assessment of cracking/ separation of the coating at this time is shown in Table 1.

Following the end of the reaction, the uncoupled heparin and residual salts were removed with water at 60°C, drying then performed for 24 hours under vacuum, and samples produced for measurement of the percentage fixed with heparin, for determining whether or not there was discoloration of the coating and for the antithrombotic test.

The determination of the percentage fixed with heparin and the evaluation as to whether or not there was coating discoloration were carried by the same methods as in Example 1, and the evaluation of the antithrombotic character was carried out as follows.

The tube was cut to a length of 8.6 cm, one side of the tube closed off with a clip, then 2.5 ml of fresh rabbit blood (blood from the rabbit carotid artery immediately after exsanguination) introduced into the middle and the other side of the tube stoppered, so as to seal-in the blood. This tube was gently shaken for 1 hour at 19°C and after 1 hour the blood was removed. After washing with physiological saline, there was introduced into the tube 3% glutaraldehyde physiological saline solution by the same method as when the fresh rabbit blood was introduced, and then the tube kept for 24 hours in a refrigerator (4°C). Subsequently, the tube was washed with water and dried for 24 hours under vacuum at room temperature. After vapour-depositing Pt-Pd on the inner face of the tube for 2 minutes, the adhering red blood cells were observed with a scanning electron microscope (SEM, Hitachi S-800), and the number adhering within an area of 445 µm x 515 µm was counted. Table 1 shows the measured percentage fixed with heparin and the results of the determination as to whether or not there was discoloration of the coating, or cracking/separation of the coating, and the number of adhering red blood cells (measured twice).

### Example 5

The same method was conducted as in Example 4 excepting that the coating of the tube interior face was carried out with a 5 wt% tetrahydrofuran solution of the graft copolymer from Example 1. Measurement of the percentage fixed with heparin, evaluation as to whether or not there was discoloration of the coating and whether or not there was cracking/separation of the coating, and measurement of the number of adhering red blood cells were carried out, and the results are shown in Table 1.

### Example 6

The same method was conducted as in Example 4 excepting that the coating of the tube interior face was carried out with a 2 wt% tetrahydrofuran solution of the graft copolymer from Example 1. Measurement of the percentage fixed with heparin, evaluation as to whether or not there was discoloration of the coating and whether or not there was cracking/separation of the coating, and measurement of the number of adhering red blood cells were carried out, and the results are shown in Table 1.

### Example 7

The same method was conducted as in Example 4 excepting that the coating of the tube interior face was carried out with a 1 wt% tetrahydrofuran solution of the graft copolymer from Example 1. Measurement of the percentage fixed with heparin, evaluation as to whether or not there was discoloration of the coating and whether or not there was cracking/separation of the coating, and measurement of the number of adhering red blood cells were carried out, and the results are shown in Table 1.

### Comparative Example 5

Coating of the tube interior face was carried out with a 2 wt% tetrahydrofuran solution of the graft copolymer of Example 1. Using this sample, evaluation as to whether or not there was discoloration of the coating and whether or not there was cracking/separation of the coating, and measurement of the number of adhering red blood cells were carried out in the same way as in Example 4. The results are shown in Table 1.

### Comparative Example 6

Using a tube which had not been coated on its inner face, the same procedure as in Example 4 was carried out. The result for the measured number of adhering red blood cells is shown in Table 1.

### Example 8

After carrying out the heparin fixing in Example 1, the tube was cut into six 6.7 cm lengths, and the ends fused together (tube surface area 20 cm²). 10 ml of rabbit serum (Gibco Laboratories Co.) was introduced in a sterile state into a 15 ml centrifuge tube (made by the Corning Co.) and then an aforesaid tube immersed. After shaking for 1 hour at 37°C (76 rpm), the tube was introduced into a separate 10 ml of rabbit serum which had previously been warmed to 37°C and shaken once again. By the same procedure there was prepared samples for measurement of the heparin elution rate after 3, 5, 8, 24 and 120 hours.

The heparin in the rabbit serum was quantitatively determined (this was carried out by the Rinsho Kensa Kikan SRL Co.) with "Test Team Heparin" (made by the Daiichi Pure Chemicals Co.) using an automatic measurement device Cobas Fara (made by Roche).

By plotting the heparin elution rate on the vertical axis and the elution time on the horizontal axis there was produce an elution curve and this is shown in Figure 1. The heparin elution maintenance time is shown in Table 1. Furthermore, using a sample following the heparin fixing, the measurement of the percentage fixed with heparin and the evaluation as to whether or not there was coating discoloration and whether or not there was cracking/ separation of the coating were carried out by the same methods as in Example 1. The results are shown in Table 1.

### Example 9

After carrying out coating with a 7 wt% tetrahydrofuran solution of the graft copolymer in Example 1, heparin fixing was carried out by the same method as in Example 1. Using the heparin-fixed tube obtained, the heparin elution rate was measured. The measurement results are shown in Figure 1 and the heparin elution maintenance time is shown in Table 1. Again, using a sample following the heparin fixing, the measurement of the percentage fixed with heparin and the evaluation as to whether or not there was coating discoloration and whether or not there was cracking/separation of the coating were carried out by the same methods as in Example 1. The results are shown in Table 1.

### Example 10

After carrying out coating with a 5 wt% tetrahydrofuran solution of the graft copolymer in Example 1, heparin fixing was carried out by the same method as in Example 1. Using the heparin-fixed tube obtained, the heparin elution rate was measured. The measurement results are shown in Figure 1 and the heparin elution maintenance time is shown in Table 1. Again, using a sample following the heparin fixing, the measurement of the percentage fixed with heparin and the determination as to whether or not there was coating discoloration and whether or not there was cracking/separation of the coating were carried out by the same methods as in Example 1. The results are shown in Table 1.

### Comparative Example 7

After carrying out coating with a 3 wt% tetrahydrofuran solution of the graft copolymer in Example 1, heparin fixing was carried out by the same method as in Example 1. Using the heparin-fixed tube obtained, the heparin elution rate was measured. The measurement results are shown in Figure 1 and the heparin elution maintenance time is shown in Table 1. Again, using a sample following the heparin fixing, the measurement of the percentage fixed with heparin and the evaluation of whether or not there was coating discoloration and whether or not there was

40 g of methyl methacrylate, 40 g of methoxypolyethylene-glycol methacrylate and 20 g of dimethylaminoethyl methacrylate were introduced into a 150 ml reaction container and dissolved in 50 g of methanol. To this solution there was added 0.1 g of V-65 (polymerization initiator) and, after uniformly mixing, nitrogen gas was blown-in over 5 minutes and a random copolymerization reaction carried out for 4 hours at 50°C. Following the end of the reaction, the reaction liquid was precipitated in methanol and the random copolymer produced was filtered off. The random copolymer was thoroughly washed with water and then dried at 40°C under vacuum for 48 hours.

The composition of this random copolymer by weight ratio was 59% methyl methacrylate, 30% methoxypolyethylene-glycol methacrylate and 11% dimethylaminoethyl methacrylate.

Next, 2 g of this random copolymer was dissolved in 9 g of tetrahydrofuran, then a solution mixture of 2.2 g of ethyl bromide and 6 g of tetrahydrofuran added thereto and a quaternizing reaction carried out for 3 hours at 50°C.

The quaternized reaction liquid was coated onto the outer face of a polyurethane tube (length 20 cm, outer diameter 1.6 mm) and left for 24 hours at room temperature. After the completion of drying, the coated tube and a 0.3 N saline solution of 2 wt% heparin were introduced into a test tube and a heparinizing reaction carried out for 72 hours at 60°C.

Following the end of the reaction, when the tube was observed it was found that cracks had been introduced in the random copolymer layer and this had separated from the tube. This result is shown in Table 1.

From the above comparative example, it is clear that the antithrombotic material of the present invention is outstanding in its antithrombotic property without discoloration or cracking/separation of the coating.

### Industrial Utilization Potential

In accordance with the present invention, there is obtained an antithrombotic medical material which is outstanding in its antithrombotic character and maintains a fixed product quality and, furthermore, in particular, no discoloration of the coating occurs.

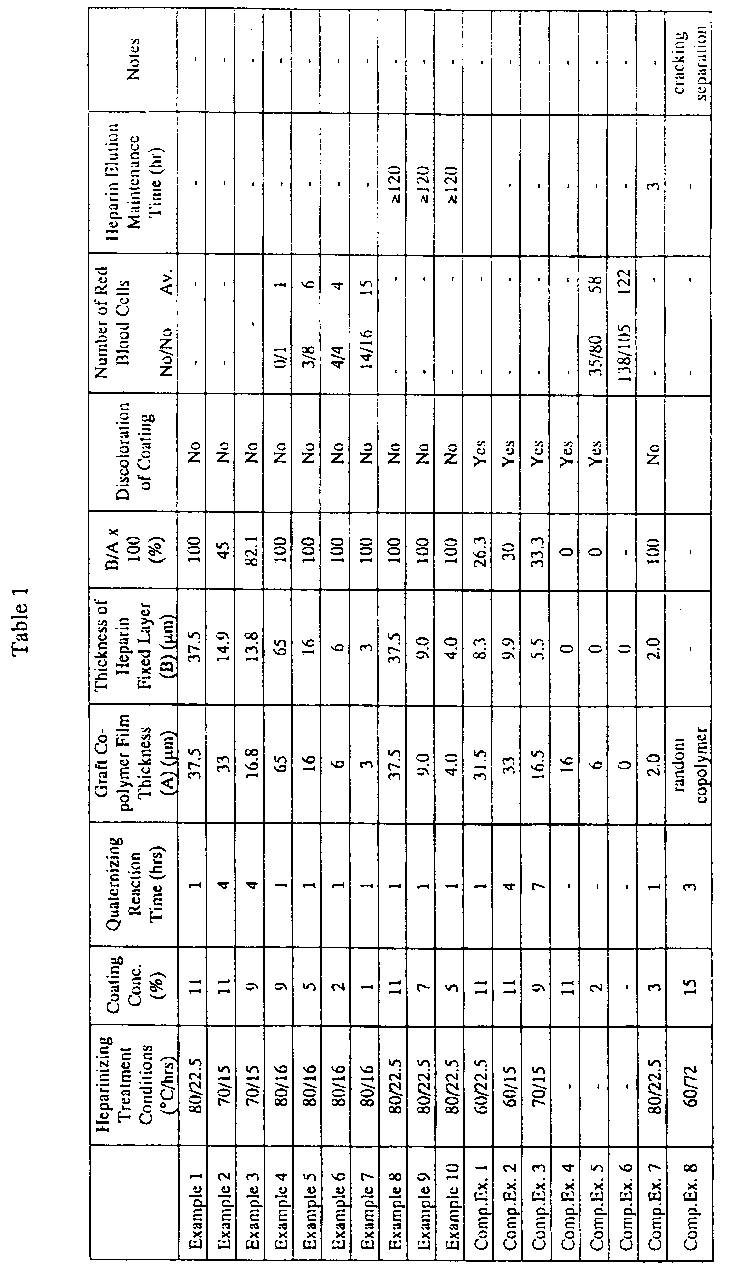

## Claims

1. An antithrombotic medical material which is characterized in that it is a medical material having, on a substrate surface, a coating comprising a graft copolymer in which monomer containing a quaternary ammonium group and hydrophilic monomer are grafted to a hydrophobic polymer, and an anticoagulant is fixed to a depth of at least 45% from said coating surface and, furthermore, the film thickness in which said anti-coagulant has been fixed is at least 3 µm.

2. An antithrombotic medical material according to Claim 1 which is characterized in that the aforesaid anti-coagulant is heparin.

3. An antithrombotic medical material according to Claim 1 which is characterized in that the aforesaid hydrophobic polymer is polyvinyl chloride.

4. An antithrombotic medical material according to Claim 1 which is characterized in that the aforesaid monomer containing a quaternary ammonium group is a vinyl compound.

5. An antithrombotic medical material according to Claim 1 which is characterized in that the aforesaid hydrophilic monomer is a vinyl compound.

6. An antithrombotic medical material according to Claim 1 which is characterized in that the aforesaid monomer containing a quaternary ammonium group is an acrylic acid derivative represented by general formula [I] 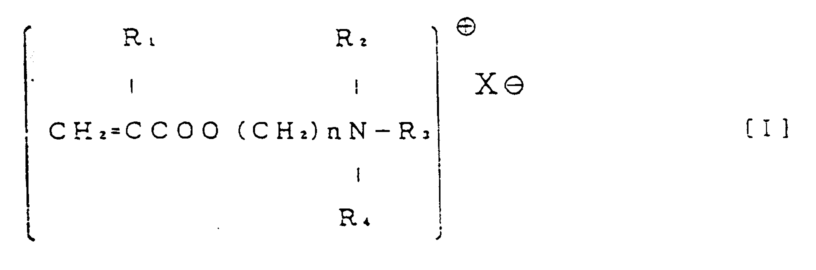 (R₁ is H or CH₃; R₂, R₃ and R₄ are H or a C₁ to C₃ alkyl group; X is a negative atomic grouping which can form a salt with amine nitrogen, and n = 2-6).

7. An antithrombotic medical material according to Claim 1 which is characterized in that the aforesaid hydrophilic monomer is an acrylic acid derivative represented by general formula [II].

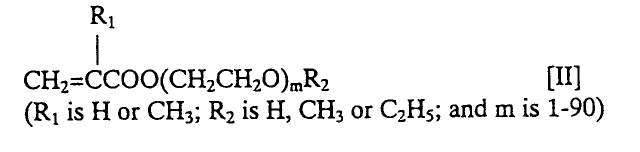

8. An antithrombotic medical material according to Claim 1 which is characterized in that it is a catheter.
